Europäisches Patentamt

European Patent Office

Office européen des brevets

(19)

(11) **EP 1 245 240 A1**

(12) **EUROPEAN PATENT APPLICATION**

(43) Date of publication:
02.10.2002 Bulletin 2002/40

(51) Int Cl.7: **A61L 24/04**

(21) Application number: 01870071.6

(22) Date of filing: **30.03.2001**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **THE PROCTER & GAMBLE COMPANY**
**Cincinnati, Ohio 45202 (US)**

(72) Inventors:
• **Goldman, Stephen Allen**
  **65013 Città Sant'Angelo, Pescara (IT)**
• **Romano, Mario**
  **65125 Pescara (IT)**

(74) Representative: **Canonici, Jean-Jacques et al**
**BVBA Procter & Gamble Europe SPRL,**
**Temselaan 100**
**1853 Strombeek-Bever (BE)**

(54) **Skin-compatible hydrogel adhesives and personal care products containing them**

(57) The present invention relates to hydrogel adhesives for attachment to mammalian skin which exhibit particularly good compatibility with skin, in addition to excellent attachment and painless removal properties including in excess moisture conditions. Such adhesives comprise 10-60 wt% of a cross-linked hydrophilic polymer, 5-80 wt% of a water-soluble nonionic humectant, and 10-85 wt% water.

The hydrophilic polymer comprises 1-50 mole% of one or more strong-acid monomers having a pKa below 3 and 50-99 mole% of one or more weak-acid monomers, having a pKa above 3, the strong-acid monomer being essentially in its salt form, and the weak-acid monomer being at least 50 mole% in its acid form, and at least 5 mole% in its salt form.

The pH of the hydrogel adhesive is from 3 to 6, preferably from 3 to 5.5, most preferably 3.5 to 5.

The present invention also encompasses personal care products containing said adhesives, such as disposable waste management articles, disposable absorbent articles, and various functional articles for attachment to the human body.

**Description**

Field of the Invention

The present invention relates to hydrogel adhesives which are capable of attaching to mammalian skin and exhibit excellent attachment and painless removal properties, including in excess moisture conditions, as well as very good compatibility with skin pH. The present invention also relates to personal care products containing the hydrogel adhesive herein, such as waste-management articles, absorbent articles, and a variety of functional articles to be worn by a human.

Background of the Invention

While hydrogel body adhesives for use in consumer products such as absorbent articles and waste-management articles have previously been described in, respectively, EP 1 025 823 and EP 1 025 866, the disclosure of hydrogel adhesive has mainly occurred in the context of medical applications, such as skin electrodes, transdermal drug delivery and wound healing. In EP 1 025 823 and EP 1 025 866, certain needs for consumer products such as absorbent and human waste-management products are disclosed, including secure attachment, painless removal and stability of adhesion in presence of excess moisture. In WO 00/46319 and WO 00/45864 are disclosed hydrogel adhesives for use in e.g. biomedical skin showing improved adhesion on wet skin and oily skin.

It has now been found that hydrogel compositions showing not only excellent attachment and painless removal properties, including in excessive moisture conditions but also excellent compatibility with the pH of skin, can be formulated. In addition, the compositions of the invention use monomers which are readily available, thus making the adhesives of the invention particularly suitable for use in consumer products manufactured in large scale.

Summary of the Invention

The present invention relates to a hydrogel adhesive for attachment to mammalian skin. Such a hydrogel adhesive comprises 10-60 wt% of a cross-linked hydrophilic polymer, 5-80 wt% of a water-soluble non-ionic humectant, and 10-85 wt% of water.

The hydrogel adhesive is characterized in that, the hydrophilic polymer comprises 1-50 mole%, preferably 5-40%, most preferably 8-30%, of one or more strong-acid monomers having a pKa below 3 and 50-99 mole%, preferably 60-95%, most preferably 70-92%, of one or more weak-acid monomers having a pKa above 3. The strong-acid monomer is essentially in its neutralized (i.e., salt) form, and the weak-acid monomer is at least 50 mole% in its acid form, and preferably at least 5 mole% in its neutralized (i.e., salt) form.

The pH of the hydrogel adhesive ranges from 3 to 6, more preferably 3 to 5.5 and most preferably 3.5 to 5.

The present invention also relates to personal care products containing the hydrogel herein.

Detailed Description

The hydrogel adhesives herein contain a cross-linked hydrophilic polymer made from two types of monomers as described hereinafter, as well as water-soluble nonionic humectant, and water. The polymerization of the monomers preferably takes place in presence of the nonionic humectant and water and the cross-linking creates a 3-dimensional matrix for the polymer, also referred to as gel form and hydrogel.

The uncrosslinked polymer includes repeating units or monomers which are selected as follows :

strong-acid monomer; the strong acid monomer is defined in relation to its pKa, which must be below 3. The pKa is measured by titration of the acid with strong base in aqueous solution according to methods well known in the art. The said strong acid monomers are preferably selected from the group of olefinically unsaturated aliphatic or aromatic sulfonic acids such as 2-acrylamido-2-methylpropanesulfonic acid, 3-sulfopropyl (meth)acrylate, 2-sulfoethyl (meth)acrylate, vinylsulfonic acid, styrene sulfonic acid, allyl sulfonic acid, vinyl toluene sulfonic acid, methacrylic sulfonic acid and the like. Particularly preferred strong-acid monomers are 2-acrylamido-2-methylpropanesulfonic acid, 3-sulfopropyl (meth)acrylate, 2-sulfoethyl (meth)acrylate.

weak-acid monomer ; the weak acid monomer is defined in relation to its pKa, which must be above 3. The said monomers are preferably selected from the group of olefinically unsaturated carboxylic acids and carboxylic acid anhydrides such as acrylic acid, methacyclic acid, maleic acid, itaconic acid, crotonic acid, ethacrylic acid, citroconic acid, fumaric acid, β-sterylacrylic acid and the like. Particularly preferred weak-acid monomers are acrylic acid and methacrylic acid.

In addition to the nature of the monomer, the respective amounts of and neutralization forms of said monomer is particularly critical in achieving the unique properties of the present hydrogel compositions.

It has been found that the level of strong acid monomer in neutralized (i.e., salt) form should not exceed 50 mole% of the hydrogel composition, and comprise between 1 and 50%, preferably 5-40%, more preferably 8-30% mole% of te composition. The level of weak-acid monomer should be equal to or greater than 50 mole% of the adhesive composition. The weak acid monomers should comprise between 50-99 mole%, preferably 60-95%, more preferably 70-92 mole% of the composition. In calculating the mole% of strong and weak acid monomers, respectively, the mole quantity per unit weight or volume of hydrogel for each type of monomer is calculated and the sum total for both strong and weak acid monomers, respectively, is divided by the sum total for all monomers. This mole fraction is then multiplied by 100 to obtain mole %.

Furthermore, it has been found that the strong acid monomer must be present essentially (e.g., at least about 99%) in its salt form and that the weak acid monomer must be present in both its acid and salt form, with the salt form representing at least about 5 mole% of said weak acid monomer and the acid form representing at least about 50 mole% of said weak acid monomer. Preferably, the weak acid monomer should be present at 60-90 mole% in acid form, and 10-40% in salt form, even more preferably from 60-80 mole% in acid form and 20-40 mole% in salt form. The acid monomers are neutralized with a suitable base, preferably neutralized with a strong base. The counterions for the neutralized (i.e., salt) forms of the weak and strong-acid monomers are preferably monovalent cations, such as $Na^+$, $K^+$, ammonium, mono/di/tri/quaternary ammonium ions. However, polyvalent cations (e.g., $Ca^{2+}$, $Al^{3+}$, polyamines and their respective quaternary ions) can also be used.

Such a careful balance contributes to achieving the required attachment force and painless removal properties, and directly influences the level of adhesion in presence of excess moisture, as well as the pH of the hydrogel composition.

<u>pH</u> :

The pH of the hydrogel composition herein is in the range of from 3 to 6, more preferably 3 to 5.5, most preferably from 3.5 to 5.5, which represents values perfectly compatible with the pH of mammalian skin.

This pH range is directly achievable by the compositions herein, without the use of any additional buffering agent, which can have a detrimental impact on the performance and skin friendliness of the hydrogels herein.

The conditions of measure of the pH are described hereinafter in the test methods section.

<u>Adhesion properties</u> : the hydrogels herein preferably have a 90° peel force on dry skin of between 0.3 to 4 N/cm, more preferably 1 to 3 N/cm. Peel force can also be measured at 180° on Polyethyleneterephthalate (PET). The hydrogels herein preferably have a peel force on PET of between 0.2-3.5 N/cm, more preferably of between 0.3-3 N/cm. The methods for measuring peel force on skin and PET are described hereinafter in the test methods section.

Furthermore, the hydrogels herein show a particularly good adhesion performance on wet-skin and in the presence of excessive moisture, such as can be found in high-humidity applications or when directly exposed to physiological fluids. A method for measuring peel force after water absorption is disclosed hereinafter.

<u>Contact Angle</u> : Without being bound by theory, it is believed that decreased hydrophilicity of the hydrogel surface improves the adhesion of the hydrogel, especially to hydrophobic surfaces such as skin. It is believed that the ability of the hydrogel to spread onto and into the interstices of the skin surface is aided when the surface energy of the hydrogel is closer to the surface energy of the skin. It is believed that decreased surface hydrophilicity of the hydrogel is particularly important for less-deformable and less-flowable hydrogels having relatively higher values of storage modulus and relatively lower values of tan δ (as described below), rheological properties that are desirable for improving the cohesiveness of the hydrogel and reducing the residue left behind on the surface (e.g., skin) when the hydrogel adhesive is repositioned and/or detached after use.

It is also believed that an increased hydrophobicity of the hydrogel surface reduces the rate of absorption of water when the hydrogel is exposed to high humidity environments and increases the capability of the hydrogel to retain its adhesion properties when it does absorb some water in a high humidity environment and/or when the hydrogel comes into direct contact with physiological fluids. It is further believed that the initial contact angle of a droplet of water on the surface of the hydrogel provides a measure of the hydrophilicity of the hydrogel surface, with a higher contact angle indicating a less hydrophilic and more hydrophobic surface.

Without being bound by theory, it is believed that, for the purpose of increasing the contact angle, the partially-neutralized weak-acid monomers of the present invention are particularly useful when combined with strong-acid monomers in their neutralized form. These weak-acid monomers contribute to a high contact angle by their ability to transfer protons from acid-form weak-acid monomers within the bulk of the hydrogel to salt-form weak-acid monomers on the surface, thus decreasing the hydrophilicity of the surface. This decrease in surface hydrophilicity is achieved without the need to concomitantly decrease the bulk hydrophilicity of the hydrogel (e.g., by decreasing the mole % of strong acid monomers in salt form and/or incorporating significant concentrations of non-ionic monomers.) Such a decrease in bulk hydrophilicity can be detrimental to other desirable properties of the hydrogel, such as its compatibility to skin, its ability to maintain a favorable pH, its ability to absorb water for adhering to wet skin, etc.

Contact angle is measured by depositing a droplet of distilled water on the surface of the hydrogel and measuring the initial angle of the drop with respect to the surface using the optical method and calculational methods described hereinafter in test methods. In determining the contact angle of the hydrogel, it is important that the measurement not be influenced by the deposition of a surface layer of hydrophobic material on the hydrogel. Such a surface layer could, for example, be transferred from a siliconized release paper applied to the exposed surface of the hydrogel.

Hydrogels of this invention preferably have a contact angle of at least 40 degrees, more preferably at least 50 degrees, even more preferably at least 60 degrees, even more preferably at least 70 degrees and most preferably at least 90 degrees.

Humectant :

The 3-dimensional adhesive matrix also comprises a humectant or mixture of humectants (also referred herein as a plastisizer), which is preferably a liquid at room temperature. The humectant is selected such that the monomer and polymer may be solubilized or dispersed within. For embodiments wherein irradiation cross linking is to be carried out, the humectant is desirably irradiation cross linking compatible such that it does not significantly inhibit the irradiation cross linking process of the polymer. The components of the humectant mixture are preferably hydrophilic and miscible with water.

Suitable humectants include alcohols, polyhydric alcohols such as glycerol and sorbitol, and glycols and ether glycols such as mono- or diethers of polyalkylene glycol, mono- or diester polyalkylene glycols, polyethylene glycols (typically up to a molecular weight of about 600), glycolates, glycerol, sorbitan esters, esters of citric and tartaric acid, imidazoline derived amphoteric surfactants, lactams, amides, polyamides, quaternary ammonium compounds, esters such as phthalates, adipates, stearates, palmitates, sebacates, or myristates, glycerol esters, including mono/di/tri-glycerides, and combinations thereof. Particularly preferred are polyhydric alcohols, polyethylene glycol (with a molecular weight up to about 600), glycerol, sorbitol and mixtures thereof. Glycerol is especially preferred. The humectant comprises 5-80 wt% of the hydrogel.

An important function of the humectant is to reduce the water activity of the hydrogel to 0.35-0.95, preferably 0.4-0.85, most preferably from 0.45-0.75. Water activity is determined by measuring the equilibrium relative humidity above the hydrogel according to the method described hereinafter in the test methods section.

Other common additives known in the art such as polymerization inhibitors, chain transfer agents, salts, surfactants, soluble or dispersible polymers, buffers, preservatives, antioxidants, pigments, mineral fillers, and the like and mixtures thereof may also be comprised within the adhesive composition in quantities up to 10 % by weight each respectively.

Other suitable monomers can also be incorporated at amounts up to about 10 mole% of the polymer. These monomers can be selected from nonionic, zwitterionic, or cationic monomers known to those skilled in the art. Examples of nonionic monomers include N,N-dimethylacrylamide, acrylamide, N-isopropyl acrylamide, hydroxyethyl (meth)acrylate, hydroxypropyl (meth)acrylate, alkyl (meth)acrylates, N-vinyl pyrrolidone and the like. Examples of cationic monomers include N,N,-dimethylaminoethyl (meth)acrylate, N,N-dimethylaminoethyl (meth)acrylamide and the respective quaternary salts and the like.

According to the present invention the polymer component of the adhesive can be physically, chemically or ionically cross linked in order to form the 3 dimensional matrix. Physical cross linking refers to polymers having cross links which are not chemical covalent bonds but are of a physical nature such that for example there are areas in the 3 dimensional matrix having high crystallinity or areas having a high glass transition temperature or areas having hydrophobic interactions. Chemical cross linking refers to polymers which are linked by chemical bonds. The polymer can be chemically cross linked by radiation techniques such as UV-, E beam- , gamma or micro-wave radiation or by copolymerizing the monomers with a di/poly-functional crosslinker via the use e.g., of UV, thermal and/or redox polymerization initiators.

Suitable polyfunctional monomer crosslinkers include polyethyleneoxide di(meth)acrylates with varying PEG molecular weights, IRR280 (a PEG diacrylate available- from UCB Chemical), trimethylolpropane ethoxylate tri(meth)acrylate with varying ethyleneoxide molecular weights, IRR210 (an alkoxylated triacrylate; available from UCB Chemicals), trimethyolpropane tri(meth)acrylate, divinylbenzene, pentaerythritol triacrylate, pentaeythritol triallyl ether, triallyl amine, N,N-methylene-bis-acrylamide and other polyfunctional monomer crosslinkers known to the art. Preferred monomer crosslinkers include the polyfunctional diacrylates and triacrylates

The monomers of the present invention are preferably polymerized via the use of a free radical polymerization initiator. Such free-radical polymerization initiators are well known in the art and can be one or more photoinitiator(s), thermal initiator(s), or redox initiator(s) and be present in quantities up to 5 % by weight, preferably from 0.02 % to 2 %, more preferably from 0.02 % to 0.4 %. Photoinitiators are preferred. Suitable photoinitiators include type I-$\alpha$-hy-

droxyketones and benzyldimethyl-ketals e.g. Irgacure 651 (dimethoxybenzylphenone; available from Ciba Specialty Chemicals) which are believed, on irradiation with UV frequencies, to form benzoyl radicals that initiate polymerization. Particularly preferred photoinitiators include 2-hydroxy-2-methyl-propiophenone (available under the trade name of Darocur 1173 from Ciba Specialty Chemicals), I-hydroxycyclohexylphenylketone (available under the trade name Irgacure 184 from Ciba Speciality Chemicals), and 4-(2-hydroxyethoxy)phenyl-(2-hydroxy-2-methylpropyl) ketone (available under the trade name of Irgacure 2959 from Ciba Specialty Chemicals). Suitable thermal initiators include potassium persulfate and VA044 (available from Wako). Suitable redox initiators include the combination of hydrogen peroxide and ascorbic acid and sodium persulfate and ascorbic acid.

Chemical crosslinking can also be effected after polymerization by use of polyfunctional reagents capable of reacting with polymer functional groups such as ethyleneglycol diglycidyl ether, polyols such as glycerol, and other polyfunctional reagents known to the art.

Crosslinking can also be effected all or in part by ionic crosslinking wherein groups of opposite charge interact via ionic interactions. Suitable ionic crosslinking agents include those known to the art including polyvalent cations such as $Al^{+3}$ and $Ca^{+2}$, di/poly-amines, di/poly-quaternary ammonium compounds, including polymeric polyamines and polyquaternary ammonium compounds known to the art.

In preparing adhesive compositions in accordance with the invention, the ingredients will usually be mixed to provide a reaction mixture in the form of an initial pre-gel aqueous based liquid formulation, and this is then converted into a gel by a free radical polymerisation reaction as described above. This may be achieved for example using conventional thermal initiators and/or photoinitiators or by ionizing radiation. Photoinitiation is a preferred method and will usually be applied by subjecting the pre-gel reaction mixture containing an appropriate photoinitiation agent to UV light after it has been spread or coated as a layer on siliconised release paper or other solid or porous substrate. The incident UV intensity, at a wavelength in the range from 240 to 420nm is of sufficient intensity and exposure duration (e.g., 10-3000 mW/cm2) to complete the polymerization in a reasonable time. To facilitate the process, it is often preferable to expose the reaction mixture to several UV irradiation sources, in sequence. The processing will generally be carried out in a controlled manner involving a precisely predetermined sequence of mixing and thermal treatment or history.

The total UV irradiation time should preferably be less than 300 seconds, more preferably less than 60 seconds, and even more preferably less than 10 seconds to form a gel with better than 95% conversion of the monomers, preferably more than 99.9% of monomers, even more preferably more than 99.99% of monomers. Those skilled in the art will appreciate that the extent of irradiation will be dependent on the thickness of the reaction mixture, reactivity and concentration of the monomers, concentration of photoinitiator, properties of the humectant, and nature of substrate on to which the reaction mixture is coated and the source of UV.

These timings are for high pressure mercury arc lamps as the source of UV operating at 200 W/cm. The peak intensity of UV reaching the surface of the substrate is approximately 1000 mW/cm$^2$. For a given lamp, the UV intensity is a function of the operating power and distance of the reaction mixture from the UV source. Also, a high-pass UV filter can be employed to minimize exposure to UV intensities of very-low wave length.

In order to minimize and preferably eliminate the presence of any residual monomers it is important to ensure that the reaction is complete. This is dependent upon a number of factors such as the substrate onto which the adhesive is applied, the type and intensity of the ultra violet light and the number of ultra violet light passes.

Rheology :

The viscous behaviour of the adhesive can be interpreted to represent an indication of the ability of the adhesive to quickly attach and securely adhere to a particular surface. The elastic behaviour can be interpreted as an indication of the "hardness" behaviour of the adhesive. Its value is also important for good initial attachment. Their combination is believed to be an indicator of the required force upon removal. The relation between elastic and viscous modulus is considered to be an indication on which fraction of the removal energy will be dissipated within the adhesive and which fraction is available to trigger the actual removal.

In order to provide adhesives for secure initial and prolonged attachment and easy/painless removal, the relation between the elastic modulus and the viscous modulus as well as their dynamic behaviour is also of importance. While not being bound by theory, it is believed that for hydrogels applied to skin, the rheological properties at T=37°C are most relevant to adhesion and removal properties. However, for the hydrogels of this invention, it has been found that the rheology properties are only at most moderately sensitive to temperature in the range of 25-37°C. Thus, for the purpose of this invention, it is convenient to specify the rheological properties at a temperature of 25°C . The adhesive has an elastic modulus at a temperature of 25°C abbreviated $G'_{25}$, a viscous modulus at a temperature of 25°C of $G''_{25}$, and the ratio of $G''_{25} / G'_{25}$ at 25°C, referred to as tan $\delta_{25}$.

The adhesive according to the present invention preferably satisfies the following conditions;

$G'_{25}$ (1 rad/sec)    is in the range 400 Pa to 20000 Pa, more preferably 700 Pa to 15000 Pa, even more preferably 1000 Pa to 10000 Pa, most preferably 2000-6000 Pa.

$G''_{25}$ (1 rad/sec)    is in the range 100 Pa to 15000 Pa, more preferably 100 Pa to 10000 Pa, even more preferably 300 Pa to 5000 Pa, most preferably 1000-4000 Pa.

and $\tan \delta_{25}$ (1 rad/s) is preferably in the range of 0.1 to 1.0, more preferably in the range of 0.3 to 0.75, even more preferably in the range of 0.35-0.65.

Provided the above rheological conditions are satisfied, the adhesives will also satisfy conditions such as sufficient cohesiveness (to prevent residue of adhesive on the skin) which are important for commercial use of such adhesives and apparent to those skilled in the art. Adhesive compositions which satisfy the above criteria can be used as adhesives for the article provided they also satisfy the common requirements of being safe for use on human or animal skin during use and generally after disposal of the article.

Often the criteria of hygienic appearance such that adhesive compositions which are transparent or white upon application are preferred.

It has been determined that the relation between the thickness or caliper C, measured in millimeters (mm), of the layer in which the adhesive is provided, typically onto at least a portion of the wearer facing surface of the article, and the viscous modulus $G''_{25}$ at about 100 rad/sec of the adhesive, is relevant to the scope of providing an easy and painless removal from the wearer's skin of such a adhesive applied on at least a portion of the wearer facing surface of an absorbent article for attachment of said article to the skin of a wearer.

The adhesive of the present invention is thus preferably provided as a layer having a thickness C such that the viscous modulus $G''_{25}$ (100 rad/sec) and the thickness C preferably satisfy the following empirical equation:

$$G''_{25} \leq [(7.00 + C) \times 3000] \text{ Pa}$$

and preferably also the following empirical equation:

$$G''_{25} \leq [(5.50 + C) \times 1700] \text{ Pa}$$

Personal Care Products

For the purpose of the present invention, personal care products means products, disposable or reusable, which are designed to be worn by a human in contact or close proximity to the body in order to achieve a function directed to the person's heath, well-being, comfort or pleasure.

A first type of such articles includes disposable, human waste management devices such as urine, menstrual and faecal management devices.

Disposable Waste-Management Devices

Urine, menstrual or faecal management devices herein include bags having an aperture and a flange surrounding the aperture for adhesive attachment to the uro genital area and or the perianal area of a wearer, Any faecal, menstrual or urine management device known in the art can be provided with an adhesive according to the present invention. Such devices are described for example in WO 99/00084 and WO 99/00085.

The urine, menstrual or faecal management devices herein also includes devices designed to be attached to artificial apertures in the body, such as ostomy/ colostomy devices.

The bag as used in such articles is a flexible receptacle for the containment of urine, menstrual and excreted faecal matter.

The bag is designed to safely contain any entrapped material, typically it will be liquid impermeable, yet it may be breathable. The bag is designed of sufficient strength to withstand rupture in use, also when pressure on the bag is exerted in typical wearing conditions, such as sitting.

The bag may contain absorbent material. The absorbent material may comprise any absorbent material which is capable of absorbing and retaining liquids. The absorbent material may comprise a wide variety of liquid-absorbent materials commonly used in disposable diapers and other absorbent articles such as comminuted wood pulp, which is generally referred to as airfelt.

The human waste management device in particular urine/menstrual management devices according to the present invention may also comprise an additional acquisition layer. The acquisition layer is typically secured to the inner surface of bag. However, the acquisition layer may also be secured to the flange, or both the flange and the inner surface of bag. The acquisition layer is preferably positioned such that it separates the genitalia of the wearer from coming into direct contact with the absorbent material. The acquisition layer is fluid pervious allowing urine/menses to readily pass through so that it may be absorbed by absorbent material.

The bag is provided with an aperture whereby excreted matter is received from the body prior to storage within the bag cavity. The aperture is surrounded by a flange and may be provided in any shape or size, such as circular, oblong, heart shaped and may be symmetrical or asymmetrical, preferably the aperture has an oblong configuration either in the longitudinal or in the transversal direction or in both directions, e.g. the contours of the aperture are in the shape of two ellipses with the respective main axes being substantially perpendicular.

The flange comprises a garment facing surface and a wearer facing surface. In an preferred embodiment these are two large, substantially flat surfaces, however, the flange may also comprise projections designed to fit the perineal or coccygeal area of the wearer.

The flange should be made of soft, flexible and malleable material to allow easy placement of the flange to the perianal area. Typical materials include nonwoven materials, wovens, open celled thermoplastic foams, closed-cell thermoplastic foams, composites of open celled foams and stretch nonwoven, and films. A closed-cell foam of polyethylene has been found effective, but more preferably an open celled polyurethane foam is used. Preferably, such foams have a thickness within the general range of 0.1 to 5 millimetres and a basis weight of 5 to 250 g/m$^2$, more preferably 50 g/m$^2$. Other thermoplastic foam materials, or other suitable plastics sheet materials having the described properties of such foams (i.e., softness, pliability, stretchability, and contractability) might also be used.

The adhesive can be applied to the wearer facing surface of the flange by any means known in the art such as slot coating, spiral, or bead application or printing. Typically the adhesive is applied at a basis weight of from 20g/m$^2$ to 2500g/m$^2$, more preferably from 500g/m$^2$ to 2000g/m$^2$ most preferably from 700g/m$^2$ to 1500g/m$^2$ depending on the end use envisioned. For example, for faecal management devices to be used for babies the amount of adhesive may be less than for faecal management devices designed for active adult incontinence sufferers.

Disposable Absorbent Articles

Another type of personal care articles herein include disposable absorbent articles such as diaper, sanitary napkins, pantiliners, tampons, perspiration pads. Absorbent articles can be made by any of the ways usual in the art. The application of the adhesive to the wearer facing surface, typically the topsheet surface of an absorbent article should not cause major problems to those skilled in the art since it can be provided by any well known techniques commonly used to apply adhesives. Most preferably the adhesive is provided in a pattern of small incremental areas such as dots or similar.

This invention can be used beneficially on disposable absorbent articles which are applied directly to the skin of a user. The article usually exhibits absorbency for bodily fluids, the protection of the user's garments from soiling, is comfortable to the user, and is easy to produce and to package. The disposable absorbent article is described below by reference to a sanitary napkin or catamenial, however diapers, panty liners, adult incontinence articles, tampons or perspiration pads are also included under the term disposable absorbent articles.

Other Personal Care Products

The present invention the adhesive herein may also find application to other personal care products. The adhesives may for example find utility to adhere functional articles which adhere to the skin such as cosmetic or pharmaceutical delivery articles which provide a substance to the skin such as skin treatment substances, cream, lotions, hormones, vitamins, deodorants, drugs; cosmetic or pharmaceutical delivery articles provide a substance to emanate away from the skin such as insecticides, inhalation drugs, perfumes and; functional articles which are not necessarily attached to the skin, but which require a high residence time on the skin such as decorative cosmetics, (lipstick, eye shadow, stage make-up) and cleaning articles (hand cleaners, face masks and hygienic pore cleansers). Such articles are preferably non-absorbent for bodily liquids.

The adhesive may also in addition find application to attach articles to the skin such as protective articles such as genital-, knee- or elbow-protectors or bandages; clothing such as bras, surgical gowns, or parts of garments during fitting at a tailor; nasal plasters; prosthesis such as breast replacements or wigs; cold wraps e.g. to provide pain relief from bruises and to reduce swelling; thermal wraps comprising thermal cells as disclosed for example in WO 97/36968 and WO 97/49361 to provide relief of temporary and chronic pain such as neck wraps as disclosed in for example US 5 728 146, knee wraps exemplified in WO 97/01311, and back wraps as disclosed for example in US 5 741 318; hearing aids; protective face masks (for the reduction or prevention of inhalation of noxious substances); anti-snoring patches, ornamental articles such as jewelry, earrings, guises, tattoos; goggles or other eye wear, tapes, bandages, dressings of general-utility, wound healing and wound management devices; and biomedical skin electrodes such as ECG, EMG, EEG, TENS electrosurgery, defibrillation, EMS and electrodes for facial/beauty applications; and fixation products and/or devices intended to affix patient catheters, tubing leadwires cables etc.

**Test Methods**

1. Rheology

The rheology of hydrogels is measured at 25°C using a suitable oscillatory rheometer such as the RHEOMETRICS SR 5000 or the equivalent. A sample of thickness of approximately 1mm is placed between two insulated Parallel Plates of 25mm diameter, controlled at a temperature of approximately 25°C using a Peltier system or equivalent. A Dynamic Frequency Sweep is performed on the hydrogel in either stress or strain mode, at an applied strain within the linear elastic response of the hydrogel (e.g., up to a strain of about 10%), with measurements at discrete frequency values between 0.1 and 100 rad/sec. Results are quoted as G', G" and tan delta at frequency values of 1.0 and 100 rad/sec.

2. Peel Force on dry skin

The peel force to remove hydrogel from dry skin is measured using a suitable tensile tester, for example an Instron Model 6021, equipped with a 10N load cell and an anvil rigid plate such as the Instron accessory model A50L2R-100. Samples are cut into strips of width 25.4mm and length between about 10 and 20 cm. A non-stretchable film of length longer than the hydrogel is applied to the reverse side of the hydrogel sample (e.g., the substrate side) using double sided adhesive. A suitable film is 23μ thick PET, available from Effegidi S.p.A, 43052, Colorno, Italy. For samples with release paper, the release paper is removed prior to applying the hydrogel to the forearm and then rolling it into place using a compression weight roller to prevent air entrapment between hydrogel and skin. The roller is 13cm in diameter, 4.5cm wide and has a mass of 5Kg. It is covered in rubber of 0.5mm thickness. The free end of the backing film is attached to the upper clamp of the tensile tester and the arm is placed below. The sample is peeled from the skin at an angle of 90 degrees and a rate of 1000mm/min. The average peel value obtained during peeling of the whole sample is quoted as the peel value in N/cm.

3. Peel force on PET

Peel force to remove hydrogel from PET is measured using a suitable tensile tester, for example an Instron Model 6021, equipped with a 10N load cell and attachment for a rigid lower plate, e.g. steel, oriented along the direction of cross-head movement. Freshly produced hydrogel is stored in a closed aluminium bag or similar for at least 12 to 24 hours at room temperature before measuring. A defect free sample of at least 100mm in length is cut from the hydrogel sample. A piece of double sided adhesive, for example type 1524 from 3M Italia S.p.A, I-20090 Segrate, Italy, at least 130mm long and 25.4mm wide is stuck to the back side of the hydrogel laminate. The hydrogel is cut along the tape's outer edges. The second liner is removed from the tape and it is stuck on the rigid base plate. A strip of standard substrate material, e.g. PET of 23μ thickness and no corona treatment, is cut to about 300mm x 40mm. Suitable material would include "Cavilen-Forex" from Effegidi S.p.A, Via Provinciale per Sacca 55, I-43052 Colorno, Italy. The release liner is removed from the hydrogel and the bottom end fixed to the rigid plate by regular tape. The standard substrate is then applied onto the body adhesive using a hand roller once forward and once backward at a speed of 1000 to 5000 mm/min. The roller is 13cm in diameter, 4.5cm wide and has a mass of 5Kg. It is covered in rubber of 0.5mm thickness. The measurement is preferably performed within 10 minutes of application of the substrate.
The free end of the standard substrate is doubled back at an angle of 180 degrees and the rigid plate is clamped in the lower clamp of the tensile tester. The free end of the standard substrate is fixed in the upper clamp of the tensile tester. The peel test is performed at a speed of 1000mm/min. The initial 20mm of peel is disregarded and the average force over the remaining length is quoted as the peel force in N/cm.

4. Peel force after water absorption

To determine the effect of water gain on the peel force of the hydrogel from skin, a sample is pre-treated such that a pre-determined weight percent of water is gained by the hydrogel, prior to measurement as described in the peel force on dry skin test method section. Moderate water gains are accomplished by placing a known weight of hydrogel in an environmental chamber, for example, a Termaks model PHDO-02 or equivalent, at 38°C and 85% relative humidity and weighing at intervals until the required weight gain is achieved. For larger water gains, a known weight of water can be distributed uniformly onto the surface of the hydrogel and allowed to be absorbed. On reaching the required weight addition of water, the sample is stored in an airtight bag to equilibrate for at least 24 hours, preferably at least 48 hours, before measuring the peel force as previously described.

### 5. Initial Contact Angle

The contact angle of a sessile drop of water on hydrogel film is measured using, for example, a Krüss DSA10 (or equivalent) instrument. Immediately after deposition on the surface of the hydrogel of a drop of purified water (HPLC grade or equivalent) of approximately 10 ul volume successive images of the drop are captured electronically and approximated to the best drop shape by a dedicated software, for example, Krüss Drop Shape Analyser software. About 25 frames per second are recorded for the first 2-3 seconds. Frames acquired before drop stabilization are discarded, then the relationship between contact angle and time is extrapolated, via linear extrapolation, to Time=0 to give the "initial contact angle" of water on hydrogel ; an average of at least 3, preferably at least 5 measurements, are taken.

### 6. pH

The pH of the hydrogel is measured using an electronic pH meter, for example as supplied by Mettler Toledo, and a flat bulb electrode, for example type InLab 426, calibrated as per the manufacturers instructions. The bulb is brought into contact with the surface of the gel and the measurement is recorded after some seconds, once the value on the display is constant. The electrode is rinsed with distilled water between successive measurements.

### 7. Water Activity (Relative Humidity)

Relative humidity is measured using an electronic humidity probe, for example the Testo 650 supplied by Testo GmbH & Company, calibrated as per the manufacturers instructions. A sample of hydrogel is placed inside the measuring chamber and sealed. Measurements are preferably made at approximately 25°C. The relative humidity and temperature are displayed on the instrument and recorded when constant. This is typically between about 30 minutes and several hours. The water activity is the relative humidity divided by 100.

### 8. Vapor-Phase Absorption

This measurement is applicable to hydrogel samples with initial water activity (as measured using the method described herein) of less than 0.85. A sample of hydrogel having a basis weight of approximately 1 $kg/m^2$ is used for this measurement. If not prepared with a vapor-impervious backing, a vapor-impervious backing (e.g., PET) is attached to the underside of a hydrogel sample. The hydrogel sample is positioned exposed-size up on an electronic balance that is pre-positioned inside a humidity and temperature controlled chamber, capable of controlling the humidity to $\pm2\%$ and temperature to $\pm1C$, for example, a Termaks model PHDO-02 or equivalent), that has been pre-equilibrated at 85% RH and 38 °C. The initial weight of the hydrogel sample is recorded as well as the change in weight of the sample as a function of time. The weight of the sample is monitored for at least 16 hours or until the sample weight has reached equilibrium. The percentage weight gain of the hydrogel sample is calculated from the recorded weight gain and the initial weight of the hydrogel, after the combined weight of any substrate or film is subtracted from the weight of the hydrogel sample. The weight gain in units of $g/cm^2$ is fitted to the function:

$$W(t) = W_f * (1-\exp(-k_a t))$$

where W(t) is the weight gain of the hydrogel, $W_f$ is the weight gain of the hydrogel at equilibrium, and $k_a$ is a rate constant in units of $sec^{-1}$ that describes the kinetics of water-vapor absorption.

### **Example 1**

An approximately 58 wt% aqueous solution of 2-acrylamido-2-methyl-1-propanesulphonic acid-monosodium salt (NaAMPS) is prepared by adding approximately 52.4 parts of 2-acrylamido-2-methyl-propanesulphonic acid (Lubrizol or equivalent) to approximately 27.3 parts of an aqueous solution containing approximately 0.5 parts of phosphate buffer (KH2PO4; Aldrich), for pH control, and approximately 100 ppm of 4-methoxy-phenol (Aldrich), an inhibitor to prevent premature polymerization. To this mixture is slowly added, with stirring, approximately 20.2 parts of 50% sodium hydroxide (NaOH; Aldrich). During this addition the temperature is maintained below 35°C. Addition of NaOH is continued until the pH of the solution increases to approximately 5.0. The final solution is cooled to ambient temperature.

Approximately 22.3 parts of the NaAMPS solution and approximately 19.2 parts of acrylic acid is added to approximately 13.3 parts of distilled water. To this solution is added, with stirring and cooling, approximately 6.4 parts of 50% NaOH (Aldrich), which is sufficient to convert approximately 30 mole% of the acrylic acid to sodium acrylate.

During this addition the temperature is maintained below 35°C. After the resultant solution is cooled to ambient temperature, approximately 38.9 parts of glycerol (Agrar) is added- with stirring. The resultant solution is covered to shield it from light. The composition of this solution, including the water produced via the neutralization of the acid monomers, is given in Table 1.

Aliquots of the above monomer solution are polymerized to form a hydrogel. Prior to polymerization, approximately 0.15 parts of the polyfunctional crosslinker IRR210 (a polyoxyethylene triacrylate crosslinker from UCB) and 0.228 parts of Darocur 1173 (2-hydroxy-2-methyl-propiophenone; Aldrich) is added to approximately 100 parts of the monomer solution and dispersed and/or dissolved with stirring for at least 15 minutes. One fraction of the monomer solution is spread at a basis weight of approximately 1.0 kilograms per square meter onto siliconized release paper for example, CO.GE.SIL "Silkraft-70g" (Palazzo), that has been surface treated by wiping with a very-thin layer of Pluronic 6400 surfactant (BASF) to facilitate spreading of the solution. For handling purposes, the release paper is pre-positioned inside a 8.5 cm diameter polystyrene Petri dish. A second fraction of the monomer solution is coated at a basis weight of approximately 1.0 kilogram per square meter onto a thin, porous non-woven substrate (Fiberweb 33; Corolind PE; 33g/sqm). This nonwoven is backed by a PET film (Cavilen - Forex; 23 μm), which is attached to the non-woven by 3M 1524 double-sided adhesive. This non-woven is pre-positioned inside a suitable container, for example a 12cm by 12cm polystyrene Petri-dish. The solution is added dropwise over the surface of the non-woven and then spread by gently tilting the box from side-to-side. An IST Model # M20-1(2)-TR-SLC UV Polymer Reactor, equipped with an IST 200 ozone-free arc lamp (Spectrum Type: CKII-OF) is used to effect polymerization. A high-pass UV filter with a frequency cut-off of approximately 310 nM (UV Filter Borofloat T320 from Bedamfpurgs-technik) is positioned between the lamp and the sample to filter out low-frequency UV irradiation The monomer-coated substrate is irradiated while passing underneath the lamp on a variable-speed belt positioned approximately 13 cm underneath the lamp. The speed of the belt is set at approximately 5 meter/min. The peak output power of the lamp is measured using an UMD-1 power meter (Eta Plus Electronic) and the output intensity of the lamp is adjusted so that the incident peak UV power on the sample is approximately 1000 milliwatt/cm$^2$. Twelve consecutive passes of the sample underneath the lamp is used to polymerize the monomer solution and convert it into a soft adhesive hydrogel. The above procedure is repeated to obtain several samples of hydrogel on each type of substrate.

The resultant hydrogels are analyzed according to the test methods described above. From samples polymerized on release paper, a 25 mm diameter punch is used to obtain a circular sample of hydrogel for measurement of rheology properties. The values are the averages for at least three samples punched from separately-polymerized hydrogel samples on release paper. The average thickness of the sample is obtained from the plate-to-plate separation used for the rheology measurement. Several 10-12 cm by 2.54cm strips are cut from one or more hydrogel sample formed on the non-woven substrate. This strip is used to measure the peel force of the adhesive from PET and from skin. Test results (average of at least three determinations) are summarized in the Table 2. Evaluation of the hydrogel sample by expert graders indicate that the hydrogel of this example has good cohesion and leaves minimal residue on skin.

The initial contact angle of water on the surface of the hydrogel was measured on samples of hydrogel polymerized on the non-woven substrate using the procedure described in the test method section.

The water activity, pH, and value for $k_a$ of the hydrogel were measured using the procedures described in the test methods section. The results are reported in the Table 2.

As can be seen from the results in Table 2, neutralizing the acrylic acid to 30% results in an excellent peel force for attachment to skin, a high contact angle, and a pH in the range of 3 to 6, which is highly compatible with skin.

## Comparative Example 2

The general procedure described in Example 1 is followed except that the mole fraction of acrylic acid monomer is increased and the mole fraction of charged monomer decreased by keeping the acrylic acid in acid form. Approximately 25.1 parts of the approximately 58 wt% NaAMPS solution and approximately 21.6 parts of acrylic acid are added to approximately 15.8 parts of distilled water. To this solution is added approximately 37.6 parts of glycerol. The resultant solution is stirred for approximately 15 minutes and covered to shield it from light The composition of this solution, is also given in Table 1. . Prior to polymerization, approximately 0.228 parts of Darocur 1173 and 0.171 parts of IRR-210 are added per 100 parts of monomer solution, and stirred to fully disperse/dissolve for at least 15mn.

Aliquots of this solution are polymerized, yielding soft adhesive hydrogels. The resultant hydrogels are analyzed as described in Example 1. The results are summarized in Table 2.

As can be seen from the results in Table 2, the decrease in acrylic acid neutralization to 0%, which is outside the compositional range of the current invention, resulted in a decrease in pH of the hydrogel to less than 3.0.

Table 1:

| Composition of Monomer Solutions | | | | | | | |
|---|---|---|---|---|---|---|---|
| Hydrogel (Ex #) | NaAMPS (moles/kg) | Na Acrylate (moles/kg) | Acrylic Acid (moles/kg) | Water (wt%) | Glycerol (wt%) | Daracur 1173 (wt%) | IRR210 (wt%) |
| 1 | 0.56 | 0.80 | 1.86 | 27.2 | 38.9 | 0.23 | 0.15 |
| 2 | 0.63 | 0.0 | 2.99 | 26.3 | 37.6 | 0.23 | 0.17 |

Table 2:

| Test Results For Adhesive Hydrogels | | | | | | | | | | | | |
|---|---|---|---|---|---|---|---|---|---|---|---|---|
| Hydro gel (Ex #) | G' (25°C) (Pa; 1 rad/s) | G" (25°C) (Pa; 1 rad/s) | Tan δ (1 rad/s) | G' (25°C) (Pa; 100 rad/s) | G"(25°C) (Pa; 100 rad/s) | Tan δ (100 rad/s) | Peel Force PET (N/cm) | Peel Force Skin (N/cm) | Contact Angle (°) | Water Activity | pH | $k_a * 10^5$ (sec$^{-1}$) |
| 1 | 4410 | 2290 | 0.52 | 17200 | 9550 | 0.55 | 1.0 | 1.8 | 67 | 0.60 | 4.8 | 8.3 |
| 2 | 4000 | 2070 | 0.52 | 17100 | 8900 | 0.52 | 2.5 | 3.2 | 76 | 0.60 | 2.3 | 7.2 |

**Claims**

1. A hydrogel adhesive for attachment to mammalian skin comprising 10-60 wt% of a cross-linked hydrophilic polymer; 5-80 wt% of a water-soluble non-ionic humectant, and 10-85 wt% water, **characterized in that** the hydrophilic polymer comprises 1-50 mole% of one or more strong-acid monomers having a pKa below 3 and 50-99 mole% of one or more weak-acid monomers, having a pKa above 3, the strong-acid monomer being essentially its salt form, and the weak-acid monomer being at least 50 mole% in its acid form, and at least 5 mole% in its salt form.

2. A hydrogel adhesive according to Claim 1 havoing a pH which ranges from 3 to 6, preferably 3 to 5.5, most preferably 3.5 to 5.

3. A hydrogel adhesive according to Claims 1-2, wherein said hydrophilic polymer comprises 5-45, preferably 8-30, mole% of one or more strong acid monomers with pKa below 3, and 60-95, preferably 70-92, mole% of one or more weak-acid monomers with pKa above 3.

4. A hydrogel adhesive according to Claims 1-3, wherein the weak acid monomer is present at 60 to 90 mole % in its acid form and 10 to 40 mole % in its salt form, preferably 60 to 80 mole % in its acid from and 20 to 40 mole% in its salt form.

5. A hydrogel adhesive according to Claims 1-4 wherein the strong-acid monomer is selected from 2-acrylamido-2-methylpropanesulfonic acid (meth)acrylic acid 3-sulfopropyl ester), (meth)acrylic acid 2-sulfoethyl ester and the weak-acid monomer is selected from acrylic acid and methacrylic acid.

6. A hydrogel adhesive according to Claims 1-5 wherein the strong-acid monomer is 2-acrylamido-2-methylpropanesulfonic acid and the weak-acid monomer is acrylic acid.

7. A hydrogel adhesive according to Claims 1-6 wherein said water-soluble nonionic humectant is selected from polyhydric alcohols, and is preferably glycerol.

8. A hydrogel adhesive according to Claims 1-7 having a water activity which is in the range of 0.35-0.95, preferably 0.4-0.85, more preferably 0.45-0.75.

9. A hydrogel adhesive according to Claims 1-8 having an elastic modulus at a temperature of 25°C $G'_{25}$ and having a viscous modulus at a temperature of 25°C, $G'_{25}$ selected such that:

   a) $G'_{25}$ (1rad/sec) is in the range of 400 Pa to 20000 Pa, preferably 700 Pa to 15000 Pa, more preferably 1000 Pa to 10000Pa, even more preferably 2000 Pa to 6000 Pa ;

   b) $G''_{25}$ (1rad/sec) is in the range of 100 Pa to 15000Pa, preferably 100 Pa to 10000 Pa, more preferably 300 Pa to 5000 Pa, even more preferably 1000 Pa to 4000 Pa ; and

   c) the ratio $G''_{25}$ (1rad/sec)/$G'_{25}$ (1rad/sec) is in the range of 0.1 to 1, more preferably in the range of 0.3 to 0.75, even more preferably in the range of 0.35 to 0.65.

10. A hydrogel adhesive according to Claims 1-9 having a contact angle of at least 40 degrees, more preferably at least 60 degrees.

11. A hydrogel adhesive according to Claims 1-10 having a peel force on dry skin of between 0.3 to 4N/cm, preferably 1 to 3N/cm or a peel force on PET of between 0.2 to 3.5 N/cm, preferably 0.3 to 3.0 N/cm.

12. A disposable human waste management device comprising a bag, said bag having an aperture and a flange surrounding said aperture, said flange having a wearer facing surface and a garment facing surface, wherein said wearer facing surface comprises a hydrogel adhesive according to Claims 1-11.

13. An absorbent article having a wearer facing surface and a garment facing surface, wherein said wearer facing surface comprises a hydrogel adhesive according to Claims 1-11.

**14.** A functional article selected from cosmetic delivery articles, pharmaceutical delivery articles, decorative cosmetic articles, cleaning articles, protective articles, clothing, prosthesis, cold wraps, thermal wraps, hearing aids, ornamental articles, goggles and eye wear, for attachment to the skin, said article comprising a hydrogel adhesive according to Claims 1-11.

**European Patent Office**

# EUROPEAN SEARCH REPORT

Application Number

EP 01 87 0071

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 00 07637 A (COLES PETER ;MUNRO HUGH SEMPLE (GB); YASIN MOHAMMED (GB); CINELLI) 17 February 2000 (2000-02-17) * examples * * page 22, line 29 - page 23, line 27 * * claims * | 1-14 | A61L24/04 |
| D,X | EP 1 026 219 A (FIRST WATER LTD) 9 August 2000 (2000-08-09) * examples * * claims * | 1-14 | |
| D,X | EP 1 025 823 A (PROCTER & GAMBLE) 9 August 2000 (2000-08-09) * page 17, line 35 - last line * * paragraph '0066! - paragraph '0073! * * claims * | 1-14 | |
| D,X | EP 1 025 866 A (PROCTER & GAMBLE) 9 August 2000 (2000-08-09) * paragraph '0060! - paragraph '0072! * * paragraph '0124! * * claims * | 1-14 | TECHNICAL FIELDS SEARCHED (Int.Cl.7) A61L C09J |
| X | WO 00 45864 A (MUNRO HUGH SEMPLE ;FIRST WATER LTD (GB)) 10 August 2000 (2000-08-10) * page 18, line 4 - page 19, line 17 * * page 27, line 12 - page 28, line 24 * * examples * * claims * | 1-11,13,14 | C08F |
| X | WO 00 07638 A (MUNRO HUGH SEMPLE ;FIRST WATER LTD (GB); YASIN MOHAMMED (GB)) 17 February 2000 (2000-02-17) * examples * * claims * | 1-11,13,14 | |

-/--

The present search report has been drawn up for all claims

| Place of search | Date of completion of the search | Examiner |
|---|---|---|
| THE HAGUE | 3 August 2001 | Thornton, S |

**European Patent Office**

## EUROPEAN SEARCH REPORT

Application Number

EP 01 87 0071

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 92 05810 A (MEDTRONIC INC) 16 April 1992 (1992-04-16) * example 1 * * claims * | 1-11,13, 14 | |
| X | DATABASE WPI Section Ch, Week 199433 Derwent Publications Ltd., London, GB; Class A12, AN 1994-269661 XP002173921 & JP 06 200224 A (NITTO CHEM IND CO LTD), 19 July 1994 (1994-07-19) * abstract * | 1-11,13, 14 | |
| A | WO 00 45698 A (AXELGAARD MFG CO LTD) 10 August 2000 (2000-08-10) * claims * | 1-14 | |
| A | DE 41 23 889 A (SANYO CHEMICAL IND LTD) 12 March 1992 (1992-03-12) * page 2, line 37 - line 44 * * claims * | 1-14 | TECHNICAL FIELDS SEARCHED (Int.Cl.7) |

The present search report has been drawn up for all claims

| Place of search | Date of completion of the search | Examiner |
|---|---|---|
| THE HAGUE | 3 August 2001 | Thornton, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03 82 (P04C01)

**ANNEX TO THE EUROPEAN SEARCH REPORT
ON EUROPEAN PATENT APPLICATION NO.** EP 01 87 0071

This annex lists the patent family members relating to the patent documents cited in the above-mentioned European search report.
The members are as contained in the European Patent Office EDP file on
The European Patent Office is in no way liable for these particulars which are merely given for the purpose of information.

03-08-2001

| Patent document cited in search report | | Publication date | Patent family member(s) | | Publication date |
|---|---|---|---|---|---|
| WO 0007637 | A | 17-02-2000 | AU | 5180199 A | 21-02-2000 |
| | | | AU | 5180799 A | 28-02-2000 |
| | | | AU | 5180999 A | 21-02-2000 |
| | | | AU | 5181199 A | 28-02-2000 |
| | | | AU | 5181799 A | 28-02-2000 |
| | | | EP | 1100556 A | 23-05-2001 |
| | | | EP | 1100554 A | 23-05-2001 |
| | | | EP | 1100557 A | 23-05-2001 |
| | | | EP | 1104312 A | 06-06-2001 |
| | | | EP | 1100555 A | 23-05-2001 |
| | | | WO | 0006214 A | 10-02-2000 |
| | | | WO | 0007636 A | 17-02-2000 |
| | | | WO | 0006215 A | 10-02-2000 |
| | | | WO | 0007638 A | 17-02-2000 |
| EP 1026219 | A | 09-08-2000 | AU | 2306000 A | 25-08-2000 |
| | | | WO | 0046319 A | 10-08-2000 |
| EP 1025823 | A | 09-08-2000 | AU | 3220700 A | 25-08-2000 |
| | | | WO | 0045766 A | 10-08-2000 |
| EP 1025866 | A | 09-08-2000 | AU | 3220800 A | 25-08-2000 |
| | | | WO | 0045865 A | 10-08-2000 |
| WO 0045864 | A | 10-08-2000 | AU | 2306100 A | 25-08-2000 |
| WO 0007638 | A | 17-02-2000 | AU | 5180199 A | 21-02-2000 |
| | | | AU | 5180799 A | 28-02-2000 |
| | | | AU | 5180999 A | 21-02-2000 |
| | | | AU | 5181199 A | 28-02-2000 |
| | | | AU | 5181799 A | 28-02-2000 |
| | | | EP | 1100556 A | 23-05-2001 |
| | | | EP | 1100554 A | 23-05-2001 |
| | | | EP | 1100557 A | 23-05-2001 |
| | | | EP | 1104312 A | 06-06-2001 |
| | | | EP | 1100555 A | 23-05-2001 |
| | | | WO | 0006214 A | 10-02-2000 |
| | | | WO | 0007636 A | 17-02-2000 |
| | | | WO | 0006215 A | 10-02-2000 |
| | | | WO | 0007637 A | 17-02-2000 |
| WO 9205810 | A | 16-04-1992 | US | 5173302 A | 22-12-1992 |
| | | | CA | 2067798 A | 29-03-1992 |
| | | | EP | 0503009 A | 16-09-1992 |
| | | | JP | 5502239 T | 22-04-1993 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**                     EP 01 87 0071

This annex lists the patent family membersrelating to the patent documents cited in the above-mentioned European search report.
The members are as contained in the European Patent Office EDP file on
The European Patent Office is in no way liable for these particulars which are merely given for the purpose of information.

03-08-2001

| Patent document cited in search report | | Publication date | Patent family member(s) | | Publication date |
|---|---|---|---|---|---|
| JP 6200224 | A | 19-07-1994 | NONE | | |
| WO 0045698 | A | 10-08-2000 | AU | 2661499 A | 25-08-2000 |
| DE 4123889 | A | 12-03-1992 | JP | 4120112 A | 21-04-1992 |